# EUROPEAN PATENT APPLICATION

(11) **EP 4 316 464 A1**
(43) Date of publication of application: **07.02.2024**
(21) Application number: 22778223.2
(22) Date of filing: 28.03.2022
(51) Int. Cl.: A61K 9/00, A61K 31/198, A61K 31/195, A61P 5/14, A61P 5/16

(54) **LONG-LASTING RESORBABLE SUBCUTANEOUS IMPLANT WITH PROLONGED RELEASE OF PRE-CONCENTRATED PHARMACOLOGICALLY ACTIVE SUBSTANCE IN POLYMER FOR THE TREATMENT OF HYPOTHYROIDISM, AND METHOD**

(30) Priority: 29.03.2021 BR 102021006083; 29.07.2021 BR 102021014927
(71) Applicant: Peracchi, Edson Luiz, CEP: 81230-161 Curitiba (BR)
(72) Inventor: Peracchi, Edson Luiz, CEP: 81230-161 Curitiba (BR)
(74) Representative: De Pablos Riba, Juan Ramon
(86) International application number: PCT/BR2022/050110
(87) International publication number: WO 2022/204771

(57) **Abstract**

The present application for privileged use of the invention is aimed at the health sector and comprises a long-term re-absorbable subcutaneous implant with prolonged release of pre-concentrated pharmacologically active substance in polymer used to treat hypothyroidism. The present application involves a biodegradable implant with isolated triiodothyronine (T3), triiodothyronine sulfate (T3S), and thyroxine (T4), the association of T3 and T4, or association of T3S and T4 for treatment of hypothyroidism in a polymer matrix. The implant is inserted subcutaneously and offers continuous release of the active ingredient for an extended period of time. This extended release seeks to guarantee efficient, constant and prolonged serum levels of the drug for the treatment of hypothyroidism. The implant has a rod-shaped cylindrical format (1), provided with straight or rounded tips, and a length and diameter between 2 to 25 mm and 1 to 6 mm, respectively. The mixture of actives during manufacturing of the implant may be molded through pressure or heat so as not to compromise the efficacy of the drug or degrade the polymeric material used. Technique options for implant molding can be: injection molding, hot molding, compression molding, or extrusion molding.

## Description

### Field of invention

The present application for privileged use of the invention is an internal priority as part of application no. BR 10 2021 006083-2, which is aimed at the healthcare sector and comprises a long-term re-absorbable subcutaneous implant with prolonged release of pre-concentrated pharmacologically active substance in polymer used to treat hypothyroidism.

### Basic invention principles

Hypothyroidism is an endocrine disease characterized by a deficiency in the endogenous production of thyroid hormones or, less commonly, the inadequate action of these hormones on target tissues. Thyroid hormones act upon nearly all of the body's nucleated cells and are essential to normal growth, as well as regulating organ function and metabolism.

Thyroid hormones are produced and regulated through a negative feedback system. Hypothalamic thyrotrophin-stimulating hormone (TRH) is produced in the hypothalamus and stimulates the synthesis and release of the thyrotrophin hormone (TSH). The release of TSH stimulates the thyroid to synthesize and release the hormones triiodothyronine (T3) and levothyroxine (T4). Increased concentrations of the hormones T3 and T4 inhibit the synthesis and secretion of TRH and TSH. This negative feedback system generates a precise means of controlling TSH levels in the body.

The decrease in the production of thyroid hormones that results in the development of hypothyroidism may result from a reduction in the action of the hormone thyrotropin (TSH) or in stimulation of the thyroid glan due to a decrease in thyrotropin-releasing hormone (TRH).

Hypothyroidism can be classified based on three criteria: onset, level of endocrine dysfunction and severity of the respective pathology. Onset refers to the disease first appearing in the patient, whether occurring congenitally or acquired throughout the patient's life span.

The level of endocrine dysfunction associated with hypothyroidism can be divided into two categories: primary and central. Primary hypothyroidism, which accounts for more than 99% of cases, is characterized by dysfunction in the thyroid gland. Central hypothyroidism, which is relatively uncommon, can be further subdivided into secondary and tertiary hypothyroidism resulting from dysfunction in the pituitary gland or hypothalamus, respectively.

Primary hypothyroidism may develop due to several different factors, with iodine deficiency considered the most prevalent cause worldwide. The human thyroid requires iodine in order to synthesize T3 and T4. As a result, if there is an insufficient presence of iodine in the body, the production of these hormones is compromised. In regions in which iodine deficiency in the population is properly controlled, the most common cause of hypothyroidism is associated with chronic autoimmune thyroid diseases, with Hashimoto's thyroiditis considered the most prevalent. Hashimoto's thyroiditis results in destruction of thyroid tissue due to activation of the immune system, which causes the body to attack thyroid cells. Primary hypothyroidism may also be drug-induced or congenital. Primary hypothyroidism may also result from medical treatments, such as those aimed at fighting cancer, or complete or partial removal of the thyroid, a procedure known as a thyroidectomy.

When considering the severity of the disease, primary hypothyroidism can be divided into declared or subclinical hypothyroidism. Hypothyroidism is classified by measuring levels of the hormones TSH, free thyroxine (T4L) and free triiodothyronine (T3L), whenever results can be reproduced over a period of 4 to 6 weeks. Whenever a patient's respective TSH value is high and T4L and T3L values are low, they are diagnosed as having primary hypothyroidism. Whenever there are no indications of the development of pituitary or hypothalamic disease, which may correspond to secondary or tertiary hypothyroidism, respectively, and the respective value for TSH is found to be higher than the reference value while T4L and T3L levels remain normal, the resulting disease is classified as subclinical hypothyroidism. Subclinical hypothyroidism may be a sign of an ongoing autoimmune disease or may indicate recovery from a certain disease or substance abuse.

With regards to central hypothyroidism, secondary hypothyroidism is more common and the occurrence of tertiary hypothyroidism is rare. Pituitary adenoma is considered the most common cause of central hypothyroidism. Low TSH, T4L, and T3L levels may be indicative of central hypothyroidism.

Hypothyroidism causes symptoms that are easily confused with other clinical conditions, particularly in postpartum women or more elderly patients. The most commonly reported symptoms include fatigue, weight gain, cold intolerance, dry skin, hair loss, constipation, memory problems, and depression.

Certain population groups are at increased risk of developing hypothyroidism, including postpartum women, individuals with a family history of autoimmune thyroid diseases, patients that have undergone surgery or irradiation to the head and neck region or the thyroid itself, individuals with other autoimmune endocrine conditions, such as type 1 diabetes, adrenal insufficiency or ovarian failure, or other non-endocrine autoimmune diseases, such as vitiligo, celiac disease, multiple sclerosis or pernicious anemia, primary pulmonary hypertension, Down's syndrome and Turner syndrome. Additionally, hypothyroidism is more prevalent among the elderly population compared to other age groups

In countries in which the population has access to adequate levels of iodine intake, hypothyroidism affects 1 to 2% of the general population. In individuals 85 years of age or older, this rate increases to approximately 7%. In Europe, the prevalence of declared hypothyroidism ranges from between 0.2% and 5.3%. In the United States this number ranges from between 0.3% and 3.7%, depending on laboratory limits adopted for diagnosis of the disease. Hypothyroidism is approximately 10 times more prevalent in women than in men. The incidence of hypothyroidism was identified in 12.3% of participants during a study involving 1220 women in the city of Rio de Janeiro.

Proper diagnosis and treatment are essential in maintaining the quality of life of patients living with hypothyroidism. The main objective during treatment of hypothyroidism is to induce a state of euthyroidism, in which circulating levels of TSH and thyroid hormones are normal, without the recurrence of adverse symptoms and clinical symptoms of the disease. In male adults, the thyroid gland secretes approximately 85 µg of thyroxine (T4) and 6.5 µg of triiodothyronine (T3) daily. This amount of T3 is only equivalent to 20% of the T3 used by the body daily; the remaining 80% (26 µg) is derived from the peripheral conversion of T4 into T3. T3 is a biologically active hormone that effectively binds to thyroid hormone receptors and acts upon peripheral tissues. T4 is a precursor to the T3 hormone and is converted into T3 through a deiodination process. This process occurs in most tissues, mainly those of the liver and kidneys.

Treatment for hypothyroidism involves the use of medications used to supply endogenous deficiencies in thyroid hormones in cases of declared and central hypothyroidism. Medical approaches for patients diagnosed with subclinical hypothyroidism include determining whether to adhere to hormone replacement treatment or not. Treatment generally involves thyroxine replacement (T4), although triiodothyronine (T3) is the bioactive hormone that effectively binds to receptors and acts on peripheral tissues.

Replacement with T4 assumes that the peripheral conversion of T4 into T3 is sufficient in meeting demands for T3 in target tissues. A dosage of approximately 1.7 µg T4 per kg/day is used in healthy adult patients, and may be reduced to up to 1 µg T4 per kg in elderly patients. However, the optimal dosage must be analyzed on a case-by-case basis based on the patient's clinical symptoms and the results of follow-up exams, taking normal TSH reference values into consideration.

Although T4 replacement treatment is the most commonly used clinical practice, there is a portion of patients that do not respond to this treatment in an adequate manner. One of causes of such a poor response is a lack of continuity in treatment due to failures in patient adherence and *compliance.* The administering of T4, which has a long half-life, should allow the body to store this hormone through binding to carrier proteins, and ensure that there is consistently enough T4 available to be deionized into T3, a hormone that is effectively bioactive. The replacement of T4 would therefore offer a sufficient amount of carrying hormone in order to ensure that T3 consistently acts upon tissues. However, if medications are not taken regularly, replacement of T4 and, consequently, improvements in the signs and symptoms of hypothyroidism, is impaired.

Another important factor during treatment is the fact that efficacy depends on deiodination of T4 into T3 in peripheral tissues. It has been confirmed that intracellular concentration of the enzyme deiodinase, which is responsible for conversion into T3, is not identical in all tissues. Additionally, if there are any issues in the patient's body that impairs such a conversion, they may experience the consequences of insufficient levels of of this hormone in the body. It is therefore necessary to use T3 in combination with T4 for effective treatment in a portion of patients with hypothyroidism.

Many studies corroborate the need to adopt therapies that combine the administering of T3 and T4 in patients, particularly among those for which TSH values are within the limits with T4 replacement that continue to experience many adverse symptoms of the disease.

A paper entitled *"Effect of combination therapy with thyroxine (T4) and 3,5,3'-triiodothyronine versus T4 monotherapy in patients with hypothyroidism, a double-blind, randomised cross-over study"* describes a randomized, double-blind, crossover study involving 59 patients with overt hypothyroidism caused by autoimmune thyroiditis receiving stable and sufficient thyroxine (T4) treatment for a period of at least 6 months. Patients were randomly divided into two groups receiving either a tablet containing 20 µg T3 as a replacement for the usual dose of 50 µg T4 or a tablet containing 50 µg T4 for 12 weeks. A 12-week crossover was implemented after this period. At the end of the treatment period, 49% of patients stated that they preferred the combined T3 and T4 treatment, and 35% did not express a preference for one or the other. Additionally, only 15% of patients preferred T4 monotherapy.

Another study performed compared T4 and T3 monotherapy. The paper titled *"Metabolic Effects of Liothyronine Therapy in Hypothyroidism: A Randomized, Double-Blind, Crossover Trial of Liothyronine Versus Levothyroxine"* describes a randomized, double-blind, crossover study involving 14 patients with primary hypothyroidism that were already receiving T4 treatment. Patients were randomly divided into two groups, one of which received 2.5, 10 or 16 µg of T3, while the other group received 5, 10 or 33 µg of T4. Both groups received the same dose three times a day, for 6 weeks. At the end of treatment, the group receiving T3 treatment lost on average 1.8 ± 1.9 kg more than the group receiving T4 treatment. A 10.9 ± 10.0% decrease in total cholesterol and a 13.3 ± 12.1% decrease in low density cholesterol (non-HDL) was also observed, in addition to an 18.3 ± 28.6% reduction in apolipoprotein B in the group receiving T3 treatment compared to T4 group. It was therefore possible to observe that treatment with T3, when compared to treatment with T4, resulted in increased weight loss and favorable changes in patient lipid profiles, without presenting significant side effects. Based on the results for T3 monotherapy treatment, it is possible to apply this treatment to patients with hypothyroidism associated with comorbidities such as cardiovascular diseases, diabetes, dyslipidemia and/or obesity.

There is also a derivative of the T3 molecule (triiodothyronine) known as triiodothyronine sulfate (T3S). Two characteristics related to the pharmacokinetic characteristics of T3S (triiodothyronine sulfate) have aroused interest regarding its use in treating hypothyroidism. This substance is biologically inactive, but is converted into T3 (triiodothyronine) through sulfatase enzymes found in different tissues, as well as intestinal microbiota. The degradation and inactivation of T3S (triiodothyronine sulfate) results from its being converted into diiodothyronine sulfate by deiodinase-1, an enzyme for which activity is directly regulated by T3 (triiodothyronine). As a result, high levels of T3 (triiodothyronine) accelerate inactivation of T3S (triiodothyronine sulfate). This mechanism allows T3 (triiodothyronine) levels to be more precisely controlled since it protects against excess hormones by stimulating deiodinase 1 degradation. Conversely, T3S (triiodothyronine sulfate) may function as a reservoir and is converted into T3 (triiodothyronine) as needed whenever there is a hormone deficiency.

The study *"Steady-State Serum T3 Concentrations for 48 Hours Following the Oral Administration of a Single Dose of 3,5,3'-Triiodothyronine Sulfate (T3S)"* demonstrated that, after single-dose oral administration, T3S (triiodothyronine sulfate) is converted into T3 (triiodothyronine), resulting in an increase in T3 (triiodothyronine) serum levels. This increase in hormones peaks between 2 and 4 hours after administration, and a progressive return to baseline levels is observed within 8 to 24 hours. The results obtained suggest that T3S (triiodothyronine sulfate) may be used in combination with LT4 (free thyroxine) to treat hypothyroidism.

In order to further investigate the efficacy and safety of this combined treatment (T4L + T3S) for hypothyroidism over an extended period of time (75 days), Santini et al. (2009) conducted a study entitled *"Treatment of hypothyroid patients with L-thyroxine (L-T4) plus triiodothyronine sulfate (T3S). Phase II, open-label, single center, parallel groups study on therapeutic efficacy and tolerability".* The study demonstrated that the proposed therapy allows normal T3 serum levels (triiodothyronine) to be maintained and restores physiological T4L (free thyroxine) / T3L (free triiodothyronine) ratios. Furthermore, there were no reports of adverse effects in patients during the study.

T3 monotherapy or combined T3 + T4 or T3S + T4 treatment for hypothyroidism carries the disadvantage of requiring administration several times a day in order to obtain optimal results, particularly given that T3 has a short half-life. The ideal treatment, which has been proposed by some authors, would be a combination of T4 and T3 medication, the latter of which has an extended-release configuration and is absorbed slowly to prevent the administering of multiple drugs. The increase in the number of tablets and the number of times throughout a day a patient takes the drug greatly decreases adherence to the treatment, which is the main disadvantage observed in literature reporting on the use of these treatments in clinical practice.

According to the World Health Organization, therapeutic adherence is determined by the interaction between the system and the health care team, socioeconomic factors, and factors related to the patient, treatment and the disease itself. Adherence to treatment is one of the main factors related to the success or failure of a therapeutic drug approach. Often the therapeutic result is not as positive as expected due to the patient's conduct in which, for various reasons, they do not continue treatment, and the drug subsequently does not produce the expected effect.

Chronic diseases, such as, cause the patient to have to take at least one drug, often more than once a day, for an extended period of time to be able to treat the disease in question. The increase in the number of medications taken by patients per day decreases adherence to treatment by approximately 20%, and drugs used in multiple doses, such as combined T4 + T3 or T4 + T3S therapy, also decreases adherence when compared to a single dose.

The oral route of administration traditionally used to treat hypothyroidism offers the major disadvantage of low therapeutic adherence. The most effective means of increase patient adherence to treatment is to simplify medication dosages. The development of extended-release drugs has made it possible to simplify dosages for many chronic diseases, including hypothyroidism.

In addition to a low level of adherence to treatment, another disadvantage presented by the oral use of medications to treat hypothyroidism is the high potential for misuse of the prescribed medication, which increases the risks of sub- or supraphysiological doses, making patients more susceptible to the adverse effects of medications or a lack of the drug, thereby compromising their general condition during treatment.

It is thereby possible to identify a third option known as implants or bioabsorbable triiodothyronine (T3), triiodothyronine sulfate (T3S), or thyroxine (T4) pellets in isolation, or the association of T3 and T4 or T3S and T4 as a treatment for hypothyroidism. Such implants involving the sustained release of medication over a long period of time in order to treat diseases, allowing treatment to function regardless of whether or not the patient takes their medication and improving clinical symptoms.

The terms "implant" or "pellet" refer to the dosage form previously consolidated under official collections of standards for drugs and pharmaceutical substances. These devices are characterized as being solid and sterile preparations of a suitable size and shape for parenteral implantation and release active substance(s) over an extended period of time.

The terms "extended release", "slow release" or "sustained release" refer to the manner in which medications are released through the implant, which occurs continuously and gradually over an extended period of time and does not result in an immediate and concentrated release of the drug into the body.

Biodegradable or bioerodible polymers refer to a polymer that degrades in vivo and in which erosion occurs over time concomitantly with and/or subsequent to release of the respective therapeutic agent. A biodegradable polymer can be a homopolymer, copolymer, or a polymer compressing more than two polymer units. In some cases, a biodegradable polymer can include mixing two or more homopolymers or copolymers.

Biodegradable or bioerodible implants can be considered implants that contain a certain mechanism that gradually reduces their mass in order to provide a prolonged period of release. The forces involved in this mass reduction may involve cellular interaction or shear forces acting on the implant surface. Erosion and gradual dissolution of the implant's components is also possible. The terms also refer to the total degradation and absorption by the body that occurs at the site where the implants were applied, excluding the need to remove the implants at the end of the treatment.

### State of the art (Background to the invention)

The document US4957119 (*Contraceptive implant*), which refers to patent registrations aimed at resorbable implants, mentions an implant made of polymeric material that can release a contraceptive agent for a relatively long period of time when adjusted subcutaneously or locally. The implant comprises an ethylene/vinyl acetate copolymer core material that functions as a matrix for a contraceptive substance, an ethylene/vinyl acetate membrane surrounding the core material, and a contact layer at the interface of the core material and membrane that prevents separation of the core material from the membrane.

Although the respective claim refers to a resorbable implant, registration no. US4957119 uses distinct active substances, and its production is carried out by means of extrusion with a very long release period for the active substance (a minimum of 1 year), which distinguishes this device from the re-absorbable subcutaneous implant constituting the present invention.

The second registration number US9980850 (*Bioerodible contraceptive implant and methods of use thereof*) describes a bioerodible contraceptive implant and methods of use in the form of a controlled release bioerodible pellet for subdermal implantation. The bioerodible sediment provides extended release of a contraceptive agent for an extended period. Bioerosion products are water-soluble, bio-reabsorbed or offer both characteristics, thereby dispensing of the need for surgical removal of the implant.

In a manner similar to the previously cited registration, record US9980850 also makes use of distinct active substances, and the period for which the active substance is released is of considerable length (between 6 months and 4 years). Furthermore, the preferred method for manufacturing pellets is the hot melt molding process.

Observing the deficiencies and pre-existing issues with regards to the conventional treatment of hypothyroidism, the present invention aims to offer a tool that assists patients in controlling hypothyroidism and improves their clinical conditions. This invention can be used both in patients with declared hypothyroidism and those diagnosed with subclinical hypothyroidism, depending on medical conduct.

The proposed treatment functions independent of the patient's memory and commitment to correct use of the medication. Due to the fact that the treatment involves prolonged release and lower dosage levels of the active substance, it is possible to reduce adverse symptoms that may be observed as part of oral ingestion of this drug and avoid hepatic metabolism since the drug is released directly into the bloodstream through means of implants. Additionally, it is not necessary to remove implants at the end of the duration period, but rather reinsert new implants in order to maintain treatment.

### Description of figures

In order to offer a clearer understanding of the present invention, the following figures are attached to this document:
Figure 1 - Representation of the chemical structure of the substances triiodothyronine (T3), triiodothyronine sulfate (T3S) and thyroxine (T4);
Figure 2 - Dimensional design of the bioabsorbable implant with isolated triiodothyronine (T3), isolated triiodothyronine sulfate (T3S), isolated thyroxine (T4), the association of T3 and T4, or association of T3S and T4 for the treatment of hypothyroidism;
Figure 3 - Dimensional design of the non-bioabsorbable implant with isolated triiodothyronine (T3), triiodothyronine sulfate (T3S), or thyroxine (T4), association of T3 and T4, or association of T3S and T4 for the treatment of hypothyroidism;
Figure 4 - Table presenting the equivalence between doses used in a treatment with oral administration of T4 and a combined treatment of T4 taken orally and a T3 subcutaneous implant.

### Detailed description of the invention

The present application for privileged use of the invention involves a biodegradable implant with isolated triiodothyronine (T3), triiodothyronine sulfate (T3S), and thyroxine (T4), the association of T3 and T4, or association of T3S and T4 for treatment of hypothyroidism in a polymer matrix. The implant is inserted subcutaneously and has continuous release of the active ingredient for an extended period of time. This extended release seeks to guarantee efficient, constant and prolonged serum levels of the drug for the treatment of hypothyroidism.

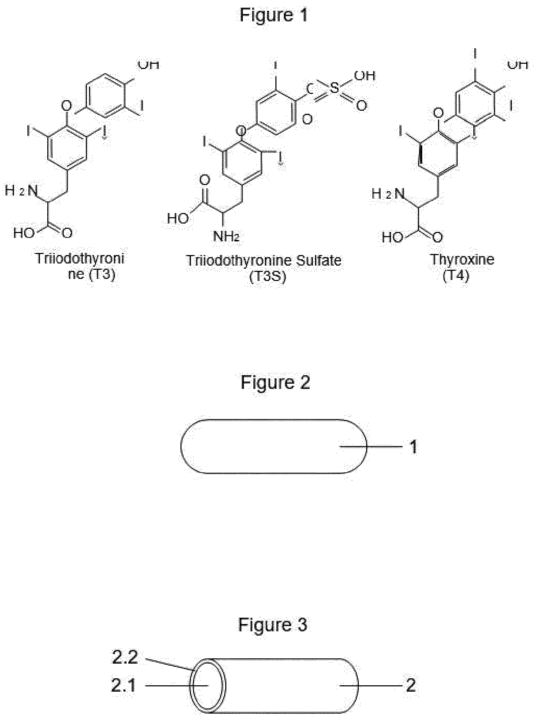

"Active substance", "active ingredient" or "drug" refers to medications used to treat hypothyroidism, which may include: triiodothyronine (T3), triiodothyronine sulfate (T3S), or thyroxine (T4) in isolation, the association of T3 and T4, or association of T3S and T4. The chemical structures of these substances are shown in figure 1.

The implant that is part of the present invention may only be constituted by the agent used to treat hypothyroidism, but is preferably formed by particles of the active substance triiodothyronine (T3), triiodothyronine sulfate (T3S), or thyroxine (T4) in isolation, the association of T3 and T4, or association of T3S and T4 homogeneously dispersed through a bioerodible and bioabsorbable polymer matrix. Such a polymer matrix may be formed of a polymer or a polymer blend. The amount of active substances present in the implant may range from between 100 and 3000 mcg of isolated T3, 100 to 3000 mcg of isolated T3S, 100 to 3000 mcg of isolated T4, a proportional association between T3 and T4, or between T3S and T4; and the substance's composition contains 1 to 20% biodegradable polymer relative to its weight.

The biodegradable polymer used may include: Poly(D-lactic acid), Poly(L-lactic acid), Poly(racemic lactic acid), Poly(glycolic acid), Poly(caprolactone), methylcellulose, ethylcellulose, hydroxypropylcellulose (HPC), hydroxypropylmethylcellulose (HPMC), polyvinylpyrrolidone (PVP), poly(vinyl alcohol) (PVA), polyethylene oxide) (PEO), polyethylene glycol, starch, natural and synthetic gum, and wax.

Implants can have any size, shape or structure that facilitates their manufacture and subcutaneous insertion, however, to obtain a more constant and uniform release of the active ingredient, it is necessary to use geometric shapes that maintain their surface area over time.

As a result, the implant developed and demonstrated as part of the present application adopts a rod-shaped cylindrical format (1), provided with straight or rounded tips, and a length and diameter between 2 to 25 mm and 1 to 6 mm, respectively. The schematic drawing of an example implant size (1) is shown in Figure 2.

Implants containing triiodothyronine (T3), triiodothyronine sulfate (T3S), or thyroxine (T4) in isolation, the association of T3 and T4, or association of T3S and T4 for the treatment of hypothyroidism may be manufactured through the addition of 100 to 3000 mcg of isolated T3, 100 to 3000 mcg of isolated T3S, 100 to 3000 mcg of isolated T4, a proportional association between T3 and T4 or a proportional association between T3S and T4 to the selected biodegradable polymer matrix solution at a proportion of 1 to 20% in relation to the drug's weight, thereby forming a homogeneous mixture. If the polymer solvent is not also one of the drug's solvent, it will be dispersed in the form of particles or as a suspension, and a *mixer* may be used to render the solution homogeneous. This solution is then dried and subsequently shaped to the shape of the implant (1) or other desired shape.

Another possible means of manufacturing the implant with triiodothyronine (T3), triiodothyronine sulfate (T3S), or thyroxine (T4) in isolation, an association of T3 and T4, or an association of T3S and T4 for the treatment of hypothyroidism involves providing a mixture of 100 to 3000 mcg of isolated T3, 100 to 3000 mcg of isolated T3S, 100 to 3000 mcg of isolated T4, a proportional association between T3 and T4 or a proportional association between T3S and T4 in each implant and at a proportion of 1 to 20% of the selected biodegradable polymer matrix relative to the weight of the drug in its dry powdered form. The drug and the polymer matrix are added in a suitable container and the mixture is homogenized.

The mixture of actives during manufacturing of the implant may be molded through pressure or heat so as not to compromise the efficacy of the drug or degrade the polymeric material used. Technique options for implant molding can be: injection molding, hot molding, compression molding, or extrusion molding.

The compression molding technique was chosen for use in the present invention. As part of this technique, a mixture of the active ingredients in powder form is added to a mold and a mechanical force is applied to the mixture, generating the compression of the particles and consequently molding the implant into the desired shape (1). The implant is then filled and sterilized with an agent used to treat hypothyroidism. The implant may be sterilized through heat (approximately 90°C) or using gamma rays.

The implant may be coated with a polymeric membrane with a thickness between 0.1 to 0.7 mm. The polymer used for the coating must be bioabsorbable and allow the passage of the active substance. The implant is preferably coatied by dipping the implant in a polymer solution. The coating may cover the entire surface of the implant including the edges, only its longitudinal surface with the edges uncoated or coated only on the edges of the implant without coating its length. The polymers that can be used for the coating are: poly(lactic-co-glycolic acid) (PLGA) and copolymers of D, L-lactic acid.

The use of non-biodegradable implants represents an additional option for treating hypothyroidism. Non-biodegradable or non-bioerodible implants (2) (figure 3) are provided with a central core (2.1) consisting of a polymer matrix at a percentage between 1 and 20% of the drug's weight, in this case 100 to 3000 mcg of isolated T3, 100 to 3000 mcg of isolated T3S, 100 to 3000 mcg of isolated T4, a proportional association between T3 and T4 or a proportional association between T3S and T4. The implant's core is surrounded by a non-degradable polymeric membrane (2.2) that controls the rate at which the drug is released.

The material used to manufacture the polymeric membrane surrounding the implant may be silicone, urethane, acrylates and related copolymers, polyvinylidene fluoride copolymers, ethylene vinyl acetate-vinyl polyethylene, and dimethylpolysiloxane. The implant's membrane has a thickness of 0.2 to 1 mm and is molded using specific equipment. After molding the membrane from the polymeric material, the active ingredient mixture is inserted, thereby forming the implant's (2) central core (2.1). The polymers used in the polymer matrix and the blend adopt the same compounds and process as the bioabsorbable implant.

The release of the drug in this system occurs through diffusion, at a relatively constant rate, and it is possible to change the rate of release of the drug through the thickness or material of this membrane. In this system, it is necessary to remove the implant at the end of the treatment.

The technological innovation subject to this patent application confers a range of possibilities for the treatment of hypothyroidism defined according to medical criteria: (a) monotherapy with triiodothyronine (T3) implants; (b) monotherapy with triiodothyronine sulfate (T3S) implants; (c) monotherapy with thyroxine (T4) implants; (d) combined T3 implant and T4 oral therapy; (e) combined T3 implant and T4 implant therapy; (f) combined T3 and T4 therapy in the same implant; (g) combined T3S implant and T4 oral therapy; (h) combined T3S implant and T4 implant therapy; (i) combined T3S and T4 therapy in the same implant.

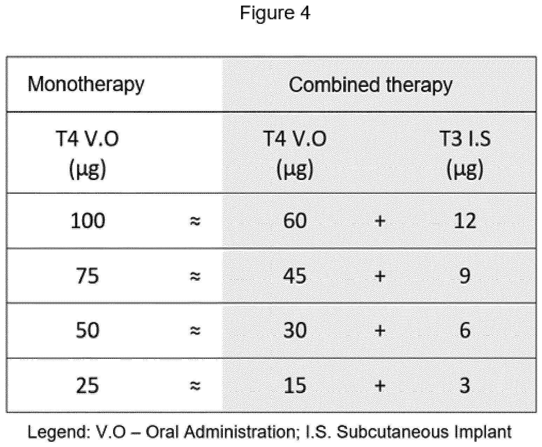

The triiodothyronine (T3) implant dosage used in the treatment of hypothyroidism is calculated based on the oral thyroxine (T4) dosage originally used. The dosage of oral T4 initially used by the patient is reduced by 40% and treatment is associated with a T3 implant with a dose equivalent to 20% of the adjusted oral dose of T4. Table 1, which is presented in figure 4, shows the equivalence between: doses used as part of an oral T4 treatment and a combined treatment of oral T4 and a subcutaneous T3 implant.

In cases involving the use of triiodothyronine (T3) associated with thyroxine (T4), or triiodothyronine sulfate (T3S) associated with thyroxine (T4), all of which are provided in the form of implants, the T4/T3 or T4/T3S ratio of 80:20 can initially be used following the physiological pattern of thyroid hormone production, or the ratio that the respective physician has defined as being most appropriate.

Regardless of the therapeutic regime used, in order to define an individualized treatment for each patient, it is necessary that medical professionals take the respective stage of hypothyroidism into account, as well as evaluate the patient's clinical condition and monitor TSH, T3L and T4L levels. In this manner, it is possible to define optimal concentration patterns and the approaches taken for each individual.

During the course of treatment, doses may be adjusted and additional implants inserted as necessary. Furthermore, if rejection or any adverse reaction occurs after insertion of the implant, it may be removed within the first days of treatment.

Use of the implant proposed herein is safe and effective as part of the treatment of hypothyroidism, given that therapy is provided independent of the patient's will or discipline in guaranteeing the action of the drug, thereby ensuring that the respective dosage and regularity of the treatment is maintained. The use of these implants as part of therapeutic management practices prevents patients from discontinuing use without medical assistance and ensures proper effective treatment is provided. Additionally, this invention prevents the patient from misusing their medication, using quantities that exceed those recommended by the physician and thereby increasing susceptibility to unwanted side effects and worsening of the patient's clinical condition.

Implants with triiodothyronine (T3), triiodothyronine sulfate (T3S), or thyroxine (T4) in isolation, the association of T3 and T4, or association of T3S and T4 for treatment of hypothyroidism helps prevents the "peaks and valleys" associated with oral administration. The implant's mechanism of action in the human body enables a more continuous release of the active substance for an extended period of time. The daily release of sufficient amounts of the drug maintains efficient serum levels, keeping blood TSH levels within normal limit and improving the patient's quality of life and treatment maintenance rates.

Combined thyroxine (T4) therapy through oral administration or an implant and a subcutaneous triiodothyronine (T3) implant and combined thyroxine (T4) therapy through oral administration or an implant and a subcutaneous triiodothyronine sulfate (T3S) implant represent an innovation in terms of the treatment traditionally applied to patients with hypothyroidism. This invention offers an alternative to patients that do not respond appropriately to T4 monotherapy since its conversion into T3 in peripheral tissues is crucial in improving the patient's symptoms and clinical condition. However, it is recognized that intracellular concentration of the enzyme deiodinase, which is converted into T3, is not identical in all tissues.

Additionally, combined therapy presents significant results for all patients undergoing treatment for hypothyroidism since it associates the bioactive hormone T3 with T4. The T3 hormone is steadily released in low doses, providing a molecule that already acts directly on tissues. T4, on the other hand, allows the body to store this hormone while bound tocarrier proteins, thereby ensuring that there is always a sufficient amount of T4 available to be deionized into T3.

Another benefit of using T3 is that it aids in weight loss and presents favorable results in patient lipid profiles, representing an option for the management of cases of hypothyroidism associated with comorbidities such as cardiovascular diseases, diabetes, dyslipidemia and/or obesity.

Implants containing triiodothyronine (T3), triiodothyronine sulfate (T3S), or thyroxine (T4) in isolation, a combination of T3 and T4, or a combination of T3S and T4 may also be used for cases of subclinical hypothyroidism, adhering to the same line of treatment used with declared hypothyroidism, whenever the respective physician deems it necessary to perform a drug intervention.

Another advantage offered by implants under this invention is due to the fact that the drug is released directly into the bloodstream, which limits side effects, renders its action much more efficient and prevents first-pass metabolism of the drug. Simplification of dosing and a decrease in frequency of administration promotes greater adherence to treatment.

## Claims

1. **LONG-TERM REABSORBABLE SUBCUTANEOUS IMPLANT WITH PROLONGED RELEASE OF PRE-CONCENTRATED PHARMACOLOGICALLY ACTIVE SUBSTANCE IN POLYMER FOR USE IN THE TREATMENT OF HYPOTHYROIDISM, CHARACTERIZED by** the biodegradable implant containing 100 to 3000 mcg of isolated triiodothyronine (T3), 100 to 30 00 mcg of isolated triiodothyronine sulfate (T3S), 100 to 3000 mcg of isolated thyroxine (T4), a proportional association of triiodothyronine (T3) and thyroxine (T4) or proportional association of triiodothyronine sulfate (T3S) and thyroxine (T4) in the form of homogeneously dispersed particles in a bioerodible and bioabsorbable polymeric matrix, with the composition of the polymeric matrix comprising 1 to 20% biodegradable polymer proportional to its total weight:

2. **LONG-TERM REABSORBABLE SUBCUTANEOUS IMPLANT WITH PROLONGED RELEASE OF PRE-CONCENTRATED PHARMACOLOGICALLY ACTIVE SUBSTANCE IN POLYMER FOR USE IN THE TREATMENT OF HYPOTHYROIDISM,** according to claim 1, **CHARACTERIZED by** use of the biodegradable polymer Poly(D-lactic acid), Poly(L-lactic acid), Poly(racemic lactic acid ), Poly(glycolic acid), Poly(caprolactone), methylcellulose, ethycellulose, hydroxypropylcellulose (HPC) hydroxypropylmethylcellulose (HPMC), polyvinylpyrolidone (PVP), poly(vinyl alcohol) (PVA), polyethylene oxide) (PEO), polyethylene glycol, starch, natural and synthetic gum and wax;

3. **LONG-TERM REABSORBABLE SUBCUTANEOUS IMPLANT WITH PROLONGED RELEASE OF PRE-CONCENTRATED PHARMACOLOGICALLY ACTIVE SUBSTANCE IN POLYMER FOR USE IN THE TREATMENT OF HYPOTHYROIDISM,** according to claims 1 and 2, **CHARACTERIZED by** the implant's rod-shaped cylindrical pattern (1) with a length between 2 and 25 mm and a diameter between 1 and 6 mm, provided with straight or rounded ends;

4. **LONG-TERM REABSORBABLE SUBCUTANEOUS IMPLANT WITH PROLONGED RELEASE OF PRE-CONCENTRATED PHARMACOLOGICALLY ACTIVE SUBSTANCE IN POLYMER USED IN THE TREATMENT OF HYPOTHYROIDISM,** according to claims 1, 2 and 3, **CHARACTERIZED by** the implant being provided with a polymeric membrane coating with a thickness between 0.1 and 0.7 mm throughout the implant, including along its edges. The implant's edges are only uncoated along its longitudinal surface or coated along the device's edges without coating along its length, using poly(acid lactic-co-glycolic acid) (PLGA) and D.L-lactic acid copolymers as a polmeric membrane;

5. **LONG-TERM REABORBABLE SUBCUTANEOUS IMPLANT WITH PROLONGED RELEASE OF PRE-CONCENTRATED PHARMACOLOGICALLY ACTIVE SUBSTANCE IN POLYMER USED IN THE TREATMENT OF HYPOTHYROIDISM,** according to claims 1 and 2, **CHARACTERIZED by** the implant being provided in a non-biodegradable or non-bioerodible form (2), with a central core (2.1) consisting of a polymeric matrix at a percentage between 1 and 20% in relation to the medication's weight, which in this case totals between 100 and 3000 mcg of isolated triiodothyronine (T3), 100 to 3000 mcg of isolated triiodothyronine sulfate (T3S), 100 to 3000 mcg of isolated thyroxine (T4), a proportional association of triiodothyronine (T3) and thyroxine (T4) or proportional association of triiodothyronine sulfate (T3S) and thyroxine (T4), with the nucleus surrounded by a non-degradable polymeric membrane (2.2);

6. **LONG-TERM REABORBABLE SUBCUTANEOUS IMPLANT WITH PROLONGED RELEASE OF PRE-CONCENTRATED PHARMACOLOGICALLY ACTIVE SUBSTANCE IN POLYMER USED IN THE TREATMENT OF HYPOTHYROIDISM,** in accordance with claims 1 and 2, **CHARACTERIZED by** the use of silicone, urethane, acrylates and their co-polymers, polyvinylidene fluoride copolymers, polyethylene vinyl acetate-ethylene vinyl acetate, polydimethylsiloxane in the material used to manufacture the polymeric membrane surrounding the implant itself. This membrane presents a thickness between 0.2 and 1 mm and, after the membrane is molded, a mixture containing active triiodothyronine is inserted, which consequently forms the implant's (2) central core (2.1).

7. **PROCESS,** according to claims 1. 2 and 3, **CHARACTERIZED by** the implant manufacturing process being initiated with the addition of 100 to 3000 mcg of isolated triiodothyronine (T3), 100 to 3000 mcg of isolated triiodothyronine sulfate (T3S), 100 to 3000 mcg of isolated thyroxine (T4), a proportional association of triiodothyronine (T3) and thyroxine (T4) or proportional association of triiodothyronine sulfate (T3S) and thyroxine (T4) in the selected biodegradable polymeric matrix solution at a proportion of 1 to 20% relative to its weight, resulting in the formation of a homogeneous mixture, which is subsequently dried and subsequent molded into the shape of the implant (1);

8. **PROCESS,** according to claims 1, 2, 3 and 7, **CHARACTERIZED by** the use of isolated triiodothyronine (T3), isolated triiodothyronine (T3S) sulfate, isolated thyroxine (T4), a combination of triiodothyronine (T3) and thyroxine (T4) or a combination of triiodothyronine sulfate (T3S) and thyroxine (T4) being used in the implant manufacturing process based on a mixture of 100 to 3000 units of the drug and 1 to 20% of the selected biodegradable polymeric matrix in relation to the drug's weight in its dry and powdered forms. These substances include isolated triiodothyronine (T3), isolated triiodothyronine sulfate (T3S), isolated thyroxine (T4), or an association of triiodothyronine (T3) and thyroxine (T4) or association of triiodothyronine sulfate (T3S) and thyroxine (T4). During the manufacturing process, the polymer matrix is added to a suitable container and the resulting mixture is homogenized;

9. **PROCESS,** according to claims 1, 2, 3, 7 and 8, **CHARACTERIZED by** the mixture of active ingredients used to manufacture the implant being pressure or heat molded. The technique selected for the manufacturing process involves compression molding in which active ingredients are mixed in powder form and added to a mold, with a mechanical force subsequently applied to the mixture, compressing the mixture's particles and molding the implant into the desired shape (1). This process is finalized with the filling and heat sterilization of the implant at 90°C or using gamma rays.
